(19) **Europäisches Patentamt**
**European Patent Office**
**Office européen des brevets**

(11) **EP 3 179 232 A1**

(12) **EUROPEAN PATENT APPLICATION**

(43) Date of publication:
**14.06.2017 Bulletin 2017/24**

(21) Application number: **15461581.9**

(22) Date of filing: **09.12.2015**

(51) Int Cl.:
*G01N 21/3577* (2014.01)     *G01N 21/359* (2014.01)
*C07C 45/33* (2006.01)     *C08G 63/00* (2006.01)
*G01N 21/552* (2014.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Purinova Sp. z o.o.**
**85-825 Bydgoszcz (PL)**

(72) Inventors:
• **Bany, Natalia**
  **85-825 Bydgoszcz (PL)**
• **Korzeniowski, Krzysztof**
  **85-825 Bydgoszcz (PL)**

• **Korzeniowski, Lukasz**
  **00-498 Warszawa (PL)**
• **Wais, Jan**
  **85-825 Bydgoszcz (PL)**
• **Piotrowski, Karol**
  **85-825 Bydgoszcz (PL)**
• **Kuklinska, Karolina**
  **85-825 Bydgoszcz (PL)**
• **Lewandowski, Marek**
  **85-825 Bydgoszcz (PL)**

(74) Representative: **Wroblewski, Michal**
**Kancelaria Patentowa**
**W.Sojka M.Wroblewski s.c.**
**ul. Gdanska 126/210**
**90-520 Lodz (PL)**

(54) **METHOD FOR DETERMINATION OF THE HYDROXYL NUMBER IN LIQUIDS HAVING AN ACID NUMBER GREATER THAN THE HYDROXYL NUMBER**

(57)     The present invention describes a method for determination of the hydroxyl number in liquids characterized by acid number value greater than the hydroxyl number using Fourier Transform Near-Infrared spectroscopy and transflective technology.

EP 3 179 232 A1

**Description**

Field of the invention

The present invention relates to a method for determining hydroxyl number in fluids wherein the acid number value is greater than the hydroxyl number, which prevents the use of a standard titration method.

Background of the invention

The hydroxyl number constitutes an important parameter that allows for the production control of many polymers, such as e.g. polyesters or polyurethanes. This number demonstrates the relationship between the molecular weight and the content of hydroxyl groups in the studied products. The increase in molecular weight results in a reduction of the hydroxyl number and a higher degree of polymerization. Traditionally, the determination of the hydroxyl number is conducted via methods based on esterification of hydroxyl groups. Well-known esterification methods involve acylation with acetic anhydride, phthalic anhydride, pyromellitic anhydride in the presence of catalysts: pyridine, 4-dimethylaminopyridine, imidazole, N-methylimidazole, perchloricacid, p-toluenesulfonicacid. In most cases, esterification is carried out in the dimethylformamide, pyridine, ethyl acetate or butyl acetate environment. Esterified groups are subjected to acid-base titration.

The publication Connors K.A .; Pandit N.K .; N Methylimidazole as a catalyst for analytical acetylations of hydroxy compounds; Anal.Chem., 1978, 50 (11), pp 1542-1545 describes acylation using an acetic anhydride in the dimethylformamide environment and in the presence of N-methylimidazole. A significant limitation to this method was an adverse impact phenolic and aldehyde groups on the result of the determination.

Then, the method for determining hydroxyl groups using pyromellitic anhydride as acylating agent is described in the publication Kingston BHM; Garey J. J .; Hellwig W. B .; Imidazole catalysis of organic hydroxyl group determination by reaction with pyromellitic dianhydride in dimethylformamide; Anal. Chem., 1969, 41 (1), pp 86-89. The determination was carried out in the dimethylformamide environment which acted as a catalyst.

Both above described methods require the use of toxic solvents, relatively long reaction time, and high temperature. Some of these limitations are not present in the method described in the Polish patent No. 152613, which recommends the use of phthalic anhydride in the presence of imidazole and acetone as the solvent. Acylation time in this case equals half an hour, while the entire determination lasts approximately ninety minutes. Determination via the method described in the Polish patent No. 162606 takes even shorter time. The use of pyromellitic anhydride as the acylating agent in the presence of N-methylimidazole in acetone made it possible to shorten the time of acylation to 5 minutes at room temperature, with a total determination time of 30 minutes.

Unfortunately, the described methods for determining the hydroxyl number are costly and involve the use of hazardous reagents. Furthermore, an important issue occurs when the acid number value is greater than the hydroxyl number. Low selectivity of titration methods does not facilitate, in this case, the correct determination of the numbers of hydroxyl groups, because a small amount of acid formed by acylation of the hydroxyl groups with an acid anhydride, is dominated by a large amount of free acids present in the sample. This reduces the ability to assess the raw material in terms of both quantity and quality.

In this context, the methods based on infrared spectroscopy, which is a fast and cheaper research technology, appear to be the alternative solution which helps to shorten the duration of quantitative analysis while reducing the use of expensive chemical reagents.

The use of Fourier Transform Infrared Spectroscopy (FTIR) for determining the hydroxyl number in polyester samples is described in Lee K. A. B; Hurley S. M .; Siepler R. A .; Mills, R. D .; Handrich K. A .; Conway J.J .; Determination of hydroxyl number in polymers by infrared spectroscopy; Applied Spectroscopy, 1990, 44 (10), pp 1719 -1721.

The FTIR method was also used to determine the presence of autoxidation products in benzenes in the publication Matuszewska A .; Odziemkowska M .; Czarnocka J .; The Use of Infrared Spectroscopy to Study Changes in Autoxidation of Fuels During Storage; Chemical Industry, 2015, 94 (8), pp. 90-94. In turn, the method for determining the hydroxyl number in polyols by ATR technique was described in S. Godoy C .; Ferrao M. F .; Gerbase A. E .; Determination of the hydroxyl value of soybean polyol Attenuated Total Reflectance to / Fourier Transform Infrared Spectroscopy; J. Amer. Oil Chem. Soc., 2007, 84, pp 503-508. The same technique for the determination of hydroxyl number in polyglycerols using the PLS (partial least squares) calibration model is described in a Polish publication from 2012: Semeniuk I .; Kosno J .; Naraniecki B .; Koreń-Szwarc B.; A New Method for Determination of Hydroxyl Number of Polyglycerols; Chemical Industry, 2012 91 (10), pp. 1952-1956.

The use of Fourier Transform Infrared Spectroscopy to determine the hydroxyl number in resins contained in toners for photocopiers and laser printers is described in U.S. Patent JP4817386B2, JP4652244B2.

Summary of the invention

The method for determination of hydroxyl groups according to the invention is devoid of the above mentioned disadvantages.

The method involves determination of the hydroxyl number in liquids which are characterized by the acid number value being greater than the hydroxyl number. The method for determination of the hydroxyl number according to the invention is particularly useful in the analysis of mixtures comprising mono acids, dicarboxylic acids and hydroxy acids which are products of, for instance, cyclohexane oxidation.

The determination is based on the use of Fourier Transform Near-Infrared (FT-NIR) spectroscopy transflective technique. It uses transflective attachments with optical path length of 1 mm and with spectral resolution of 4 $cm^{-1}$. The method according to the invention is characterized by high accuracy. The accuracy of the method is 97.5% with precision mark of 1.57. The repeatability of the method equals 0.01, and the average standard deviation is 1.04. The duration of one determination is 2 minutes.

Detailed description of the invention.

The method for determination of the hydroxyl number according to the invention is illustrated in the Examples.

Example 1. Preparation of calibration model.

The absorbance value at a particular wave number is linearly dependent on the number of hydroxyl groups in the sample. For the cyclohexane oxidation this dependence is described numerically with the use of PLS (partial least squares) calibration model. This model is prepared based on 50 samples of polyester poly-alcohol obtained from cyclohexane oxidation product (PUC) with a known hydroxyl number as determined by reference method involving acylation with acetic anhydride in the dimethylformamide environment and in the presence of catalytic amounts of 4- (N, N-dimethylamino) pyridine, hydrolysis of the excess anhydride and acid-base titration.

Hydroxyl number values of the calibration samples were in the range of 323 - 437 mg KOH/g. The model consisted of two absorption regions of 2000-2300 nm and 1380-1500 nm, which reflects the wavenumber range of 4000-10000 $cm^{-1}$. Calibration samples were analyzed in accordance with point 2. The spectrometer software was used in the calculations.

Example 2. Preparation of samples - analysis.

Petri dish with the diameter of approximately 50 mm was washed in acetone and distilled water, and then placed in a dryer at the temperature of 50° C for 60 min. At room temperature, the tested PUC sample was placed on the integrating sphere of Petri dish in order to form a 2-4 mm layer of the sample. Then, the transflective attachment with the optical path length of 1 mm was immersed in the sample in a way that prevented air bubbles from entering the space under the attachment. The sample prepared in this way was placed on the measuring cell of FT-NIR spectrometer. Analysis of the sample's hydroxyl number was performed four times using the spectrometer software under the same conditions in which the calibration model was prepared. The following results were obtained:

$$L_{OH} = 57{,}7 \text{ mg KOH/g } (x_1)$$

$$L_{OH} = 58{,}2 \text{ mg KOH/g } (x_2)$$

$$L_{OH} = 58{,}5 \text{ mg KOH/g } (x_3)$$

$$L_{OH} = 57{,}1 \text{ mg KOH/g } (x_4)$$

The average hydroxyl number value of four determinations (n = 4) equals 57.9 mg KOH/g.

Standard deviation calculated according to the formula $S = \sqrt{\dfrac{\sum (x_1 - \bar{x})^2}{n-1}}$ equals 0.61, and the relative deviation is:

$$\frac{s}{\bar{x}} = \frac{0.61}{57.9} = 0.01$$

The hydroxyl number of the sample determined on the basis of calculation = 57.3, and thus the percentage error is:

$$\frac{(57.9 - 57.3) \cdot 100}{57.3} = 1.05.$$

## Claims

1. Method for determination of the hydroxyl number in liquids **characterized by** acid number value greater than the hydroxyl number using Fourier Transform Near-Infrared spectroscopy, **characterized in that** the determination is carried out with the use of transflective technology.

2. The method according to claim 1, **characterized in that** it uses transflective attachments with optical path length of 1 mm at adopted spectral resolution of 4 cm$^{-1}$.

Europäisches
Patentamt

European
Patent Office

Office européen
des brevets

# EUROPEAN SEARCH REPORT

Application Number

EP 15 46 1581

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2007/184377 A1 (SHU HYO [JP] ET AL) 9 August 2007 (2007-08-09) * paragraphs [0028], [0046], [0090], [0093], [0095], [0096], [0132], [0133] * | 1,2 | INV.<br>G01N21/3577<br>G01N21/359<br>C07C45/33<br>C08G63/00<br>G01N21/552 |
| Y,D | JP 4 817386 B2 (RICOH KK) 16 November 2011 (2011-11-16) * abstract * * paragraphs [0014], [0066], [0067], [0115] * | 1,2 | |
| Y,D | JP 4 652244 B2 (RICOH KK) 16 March 2011 (2011-03-16) * abstract * * paragraphs [0033], [0034] * | 1,2 | |
| Y | HORMOZ AZIZIAN ET AL: "Evaluating the Transferability of FT-NIR Calibration Models for Fatty Acid Determination of Edible Fats and Oils Among Five Same-make Spectrometers Using Transmission or Transflection Modes with Different Pathlengths", JOURNAL OF THE AMERICAN OIL CHEMISTS' SOCIETY (JAOCS), vol. 89, no. 12, 7 August 2012 (2012-08-07), pages 2143-2154, XP055256725, DE ISSN: 0003-021X, DOI: 10.1007/s11746-012-2116-9 * pages 2143-215 * | 1,2 | TECHNICAL FIELDS SEARCHED (IPC)<br><br>G01N<br>C07C<br>C08G |
| Y | US 2015/293005 A1 (REIMERS JAY L [US]) 15 October 2015 (2015-10-15) * paragraphs [0002], [0021], [0051], [0052], [0057], [0077] * | 1,2 | |

-/--

The present search report has been drawn up for all claims

| Place of search | Date of completion of the search | Examiner |
|---|---|---|
| Munich | 9 March 2016 | Duijs, Eric |

EPO FORM 1503 03.82 (P04C01)

Europäisches
Patentamt

European
Patent Office

Office européen
des brevets

## EUROPEAN SEARCH REPORT

Application Number

EP 15 46 1581

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | OLIVEIRA J S ET AL: "Determination of methyl ester contents in biodiesel blends by FTIR-ATR and FTNIR spectroscopies", TALANTA, ELSEVIER, AMSTERDAM, NL, vol. 69, no. 5, 15 July 2006 (2006-07-15), pages 1278-1284, XP025000719, ISSN: 0039-9140, DOI: 10.1016/J.TALANTA.2006.01.002 [retrieved on 2006-07-15] * abstract * * page 1279 * * page 1283 - page 1284 * ----- | 1,2 | |
| | | | TECHNICAL FIELDS SEARCHED (IPC) |

The present search report has been drawn up for all claims

| Place of search | Date of completion of the search | Examiner |
|---|---|---|
| Munich | 9 March 2016 | Duijs, Eric |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 15 46 1581

This annex lists the patent family members relating to the patent documents cited in the above-mentioned European search report.
The members are as contained in the European Patent Office EDP file on
The European Patent Office is in no way liable for these particulars which are merely given for the purpose of information.

09-03-2016

| Patent document cited in search report | | Publication date | Patent family member(s) | | Publication date |
|---|---|---|---|---|---|
| US 2007184377 | A1 | 09-08-2007 | CN 101017352 A<br>US 2007184377 A1<br>US 2011002719 A1 | | 15-08-2007<br>09-08-2007<br>06-01-2011 |
| JP 4817386 | B2 | 16-11-2011 | JP 4817386 B2<br>JP 2007241241 A | | 16-11-2011<br>20-09-2007 |
| JP 4652244 | B2 | 16-03-2011 | JP 4652244 B2<br>JP 2007206097 A | | 16-03-2011<br>16-08-2007 |
| US 2015293005 | A1 | 15-10-2015 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- PL 152613
- PL 162606
- JP 4817386 B
- JP 4652244 B

**Non-patent literature cited in the description**

- **CONNORS K.A . ; PANDIT N.K .** N Methylimidazole as a catalyst for analytical acetylations of hydroxy compounds. *Anal.Chem.,* 1978, vol. 50 (11), 1542-1545
- **KINGSTON BHM ; GAREY J. J . ; HELLWIG W. B .** Imidazole catalysis of organic hydroxyl group determination by reaction with pyromellitic dianhydride in dimethylformamide. *Anal. Chem.,* 1969, vol. 41 (1), 86-89
- **LEE K. A. B ; HURLEY S. M . ; SIEPLER R. A . ; MILLS, R. D . ; HANDRICH K. A . ; CONWAY J.J .** Determination of hydroxyl number in polymers by infrared spectroscopy. *Applied Spectroscopy,* 1990, vol. 44 (10), 1719-1721
- **MATUSZEWSKA A . ; ODZIEMKOWSKA M . ; CZARNOCKA J .** The Use of Infrared Spectroscopy to Study Changes in Autoxidation of Fuels During Storage. *Chemical Industry,* 2015, vol. 94 (8), 90-94
- **S. GODOY C . ; FERRAO M. F . ; GERBASE A. E .** Determination of the hydroxyl value of soybean polyol Attenuated Total Reflectance to / Fourier Transform Infrared Spectroscopy. *J. Amer. Oil Chem. Soc.,* 2007, vol. 84, 503-508
- A New Method for Determination of Hydroxyl Number of Polyglycerols. *Chemical Industry,* 2012, vol. 91 (10), 1952-1956